# EUROPEAN PATENT APPLICATION

(11) **EP 4 364 758 A1**
(43) Date of publication of application: **08.05.2024**
(21) Application number: 22832579.1
(22) Date of filing: 20.04.2022
(51) Int. Cl.: A61L 2/18, A61L 101/22

(54) **PASS BOX FOR DECONTAMINATION**

(30) Priority: 30.06.2021 JP 2021108687
(71) Applicant: Airex Co., Ltd., Nagoya-shi Aichi 453-0015 (JP)
(72) Inventor: KAWASAKI, Koji, Nagoya-shi Aichi 453-0015 (JP); KAKUDA, Daisuke, Nagoya-shi Aichi 453-0015 (JP); KITAHORA, Kazuhiko, Nagoya-shi Aichi 453-0015 (JP); FUTAMURA, Haruka, Nagoya-shi Aichi 453-0015 (JP); OGATA, Yoshitaka, Nagoya-shi Aichi 453-0015 (JP); GUO, Zhiqiang, Nagoya-shi Aichi 453-0015 (JP); KITANO, Tsukasa, Nagoya-shi Aichi 453-0015 (JP)
(74) Representative: Kraus & Lederer PartGmbB
(86) International application number: PCT/JP2022/018283
(87) International publication number: WO 2023/276418

(57) **Abstract**

A pass box for decontamination which can achieve a complete decontamination effect at a proper temperature and by a decontamination operation in a short time without giving an influence such as a thermal quality change to a thermosensitive substance when an outer surface of a container which accommodates the thermosensitive substance is decontaminated is provided.

A pass box main-body including a carrying-in zone, a decontamination zone, an aeration zone, and a carrying-out zone and a carrying device which carries an article inside the pass box main-body are provided. A decontamination-agent applying device includes a compressed-gas generating means which generates a compressed gas, a decontamination-agent supply means which supplies a decontamination agent, a mixed gas-liquid regulator which mixes the compressed gas and the decontamination agent and regulates a mixed gas-liquid, and a heater which heats the mixed air-liquid and makes it into a decontamination agent mist. An amount of the compressed gas supplied from the compressed-gas generating means, an amount of the decontamination agent supplied from the decontamination-agent supply means, and a heating temperature of the heater are controlled, and a temperature of the decontamination agent mist applied to an outer surface of an article is controlled.

## Description

### TECHNICAL FIELD

The present invention relates to a pass box for decontamination and particularly relates to a pass box to be used, when an outer surface of a container which accommodates a thermosensitive substance inside is decontaminated, and the container is carried into a work room maintained in a sterile environment.

### BACKGROUND ART

At a manufacturing site where medicines or foods are manufactured or at a medical setting such as an operating room, it is important to maintain the sterile state in the room. Particularly in the decontamination of the sterile room, which is a work room for manufacturing the medicines, the high-level decontamination validation in compliance with the GMP (Good Manufacturing Practice) needs to be completed.

In a small-scale work under the sterile environment as above, a small-sized chamber is employed as a work room, and a worker uses an isolator which can enable a work through a glove or a half suit from an outside of this chamber. In this chamber of the isolator, a sucking/exhaust device which maintains the sterile state so that a contaminant does not mix from the outside environment is provided. Moreover, when required tools and articles are to be carried into the isolator in the sterile state from the outside environment, too, the sterile state is maintained.

For example, when an article is to be carried into the isolator, a small-sized preliminary chamber for the carrying-in called a pass box is provided. A worker who is to carry the article into the isolator first carries the article into the pass box. At this time, a carrying-in door between the isolator and the pass box is blocked. Subsequently, the carrying-in door between the pass box and the outside environment is blocked, and the article is decontaminated. After the decontamination in the pass box is completed, and a decontamination gas or the like is eliminated, the carrying-in door between the isolator and the pass box is opened, and the article is carried into the isolator.

Recently, for the decontamination of a work room such as an isolator and a pass box (hereinafter, referred to as a "decontamination target room") and an article to be carried in, a hydrogen peroxide (a gas or a mist) is widely employed. This hydrogen peroxide has a strong sterilization effect, is inexpensive and is easily available, and is effective as a decontamination gas which is finally decomposed to oxygen and water and is friendly to the environment. It is described in the Patent Document 1 that the decontamination effect by this hydrogen peroxide is caused by a condensed film of a hydrogen peroxide solution condensed on a surface of a decontamination target part.

By the way, the articles to be decontaminated in the pass box and to be carried into the isolator include a container (vials, bottles and the like) which accommodates substances which are instable to a heat (hereinafter, referred to as a "thermosensitive substance"). Here, as the thermosensitive substance, medical materials, medicines (also including biological drug products, vaccines and the like), medicines after being frozen and dried, biological cells, cells offered for regenerative medicines (including those in a frozen state), culture reagents and the like are cited. These thermosensitive substances are strictly managed at a low optimal storage temperature at which the substance is stable such as a refrigeration storage, a freezing storage and the like.

In order to decontaminate a surface of a container which accommodates a thermosensitive substance in a short time and with a high decontamination effect, a high-temperature decontamination gas or the like is preferably supplied to the container surface which accommodates the thermosensitive substance. However, it is not preferable to expose the container which accommodates the thermosensitive substance to a temperature largely deviated from the optimal storage temperature of the thermosensitive substance accommodated therein for a long time. Thus, the inventors proposed a pass box which can promote a complete decontamination effect for a low-temperature substance such as a frozen drink as a target and can promote a reduction of a work time and the efficiency of the decontamination work in Patent Document 2.

In the pass box according to the Patent Document 2, a decontamination agent mist is supplied toward the surface of the low-temperature article, and a condensed film of a decontamination agent is formed on a surface of the low-temperature article. Subsequently, in a drying process, a dry air is supplied toward the surface of the low-temperature article, an ultrasonic vibration and an acoustic radiation pressure are made to act on the condensed film, and the work time is shortened.

### CITATION LIST

### PATENT LITERATURE

[Patent Document 1] Japanese Patent Publication No. 1986-4543
[Patent Document 2] Japanese Unexamined Patent Application Publication No. 2020-157005

### SUMMARY OF INVENTION

### TECHNICAL Problem

By the way, in the aforementioned Japanese Unexamined Patent Application Publication No. 2020-157005, when a low-temperature sample as a target is to be decontaminated, the decontamination agent mist in the vicinity of a room temperature (20 to 25°C) is applied to a surface of the low-temperature sample. In this case, if a surface temperature of the low-temperature sample is within a range from 0 to 10°C, a high decontamination effect can be acquired in a relatively short time. However, in the decontamination on the surface of an extremely low-temperature sample at -10°C or below, there was a problem that a time for a contact with the decontamination agent mist needs to be longer in order to obtain a sufficient decontamination effect. Therefore, in the decontamination on the surface of a container which accommodates a thermosensitive substance whose quality change to a heat is extremely sensitive, there was a problem that an influence on the quality became grave.

Thus, in order to deal with the aforementioned problems, the present invention has an object to provide a pass box for decontamination which can promote a complete decontamination effect at a proper temperature and by a decontamination operation in a short time without giving an influence such as a thermal quality change to the thermosensitive substance when an outer surface of a container which accommodates the thermosensitive substance is decontaminated.

### SOLUTION TO PROBLEM

In solving the aforementioned problem, as the result of keen researches, the inventors have found that, when a carrying device which controls a temperature of a decontamination agent mist to be applied to a surface of an article and continuously or semi-continuously carries the article in the pass box is employed, whereby aforementioned object can be achieved and thus, the present invention was completed.

That is, a pass box for decontamination according to the present invention is, according to a description in claim 1,
a pass box used when an article is to be carried into a work room in which a sterile environment is maintained,
having a pass box main-body which has a carrying-in zone into which the article is carried from an outside environment, a decontamination zone in which an outer surface of the carried-in article is decontaminated by a decontamination agent, an aeration zone in which the decontamination agent remaining on the outer surface of the article after the decontamination is removed, and a carrying-out zone from which the article after the aeration is carried out into the work room and a carrying device which carries the article inside the pass body main-body, characterized in that
the carrying-in zone includes an airtightly sealable carrying-in port through which the article is carried in from the outside environment,
the decontamination zone includes a decontamination-agent applying device which applies the decontamination agent mist to the outer surface of the article which has been carried by the carrying device from the carrying-in zone,
the aeration zone includes a clean-air applying device which applies a clean air to the surface of the article which has been carried by the carrying device from the decontamination zone,
the carrying-out zone includes a carrying-out port through which the article is carried out by the carrying device to an inside of the work room,
the decontamination-agent applying device includes a compressed-gas generating means which generates a compressed gas, a decontamination-agent supplying means which supplies the decontamination agent, a mixed gas-liquid regulator which mixes the compressed gas and the decontamination agent and regulates a mixed gas-liquid, and a heater which heats the mixed gas-liquid and obtains the decontamination agent mist, and
an amount of the compressed gas supplied from the compressed-gas generating means, an amount of the decontamination agent supplied from the decontamination-agent supply means, and a heating temperature of the heater are controlled, whereby the temperature of the decontamination agent mist applied to the outer surface of the article is controlled.

Moreover, the present invention is, according to the description in claim 2, the pass box for decontamination described in claim 1, characterized in that
the carrying device operates in a first carrying process, a second carrying process, and a third carrying process,
in the first carrying process, a part of the article which has been carried into the carrying-in zone and has not been decontaminated is supported and is carried from the carrying-in zone to the decontamination zone, and a most part other than the part supported in the decontamination zone is decontaminated,
in the second carrying process, in a state in which a part of the part decontaminated in the first carrying process is switched to be supported and is carried from the decontamination zone to the aeration zone, and a part other than the part decontaminated in the first carrying process is decontaminated in the decontamination zone and also a most part other than the part supported in the aeration zone is aerated, and

in the third carrying process, in a state in which a part of the part aerated in the second carrying process is switched to be supported and is carried from the aeration zone to the carrying-out zone, and a part other than the part aerated in the second carrying process is aerated.

Moreover, the present invention is, according to the description in claim 3, the pass box for decontamination described in claim 2, characterized in that
the carrying device is constituted by three units of carrying devices, that is, a first carrying device, a second carrying device, and a third carrying device,
the first carrying device operates in the first carrying process,
the second carrying device operates in the second carrying process, and
the third carrying device operates in the third carrying process.

Moreover, the present invention is, according to the description in claim 4, a pass box for decontamination described in claim 2, characterized in that
the carrying device is constituted by two units of carrying devices, that is, the first carrying device and the second carrying device,
the first carrying device operates in the first carrying process and the third carrying process, and
the second carrying device operates in the second carrying process.

Moreover, the present invention is, according to the description in claim 5, a pass box for decontamination described in any one of claims 2 to 4, characterized in that
the carrying device operates continuously or semi-continuously, whereby
a total of each operation time of the first carrying process, the second carrying process, and the third carrying process is within 10 minutes.

Moreover, the present invention is, according to the description in claim 6, the pass box for decontamination described in any one of claims 1 to 5, characterized in that
a temperature of the decontamination agent mist is controlled within a range from 30 to 60°C.

Moreover, the present invention is, according to the description in claim 7, the pass box for decontamination described in any one of claims 1 to 6, including
a first exhaust device which exhausts an air in the carrying-in zone and the decontamination zone and a second exhaust device which exhausts an air in the aeration zone and the carrying-out zone, characterized in that
an exhaust amount of the first exhaust device is set larger than an exhaust amount of the second exhaust device, whereby the air in the carrying-in zone and the decontamination zone does not mix in the aeration zone and the carrying-out zone, and
an air pressure in the pass box main-body is set to a more negative pressure than an air pressure inside the work room, whereby the air in the pass box main-body does not mix inside the work.

Moreover, the present invention is, according to the description in claim 8, the pass box for decontamination described in any one of claims 1 to 7, characterized in that
the heater includes an introduction port which introduces the mixed gas-liquid, a cylindrical outer cylinder tube, a heat generating body incorporated inside the outer cylinder tube, in parallel to a longitudinal direction of the outer cylinder tube, and an ejection port through which the decontamination agent mist is ejecetd, and
the mixed gas-liquid is heated while passing between the outer cylinder tube and the heat generating body, the whole or a part of the mixed gas-liquid becomes a decontamination agent gas, and the decontamination agent gas is made into a mist by the compressed gas whose amount is controlled and changed to the decontamination agent mist whose temperature is controlled.

Moreover, the present invention is, according to the description in claim 9, the pass box for decontamination described in any one of claims 1 to 7, characterized in that
the heater includes an introduction port which introduces the mixed gas-liquid, a cylindrical outer cylinder tube, a heat generating body incorporated inside the outer cylinder tube, in parallel to a longitudinal direction of the outer cylinder tube, and an ejection port through which the decontamination agent mist is ejected,
the heat generating body includes a rod-shaped heater disposed in a longitudinal direction thereof and an evaporation tube wound in a spiral state in the longitudinal direction along an outer periphery of the heater, and
the mixed gas-liquid is heated while passing through the inside of the evaporation tube, the whole or a part of the mixed gas-liquid becomes the decontamination agent gas, and the decontamination agent gas is made into a mist by the compressed gas whose amount is controlled and is changed to the decontamination agent mist whose temperature is controlled.

Moreover, the present invention is, according to the description in claim 10, the pass box for decontamination described in claim 8 or 9, characterized in that
the heater includes a mixed gas regulator which mixes the decontamination agent gas and the compressed gas between the heat generating body and the ejection port, and
an amount of the compressed gas to be introduced into the mixed gas regulator is controlled, whereby the decontamination agent gas is made into a mist by the compressed gas and is changed to the decontamination agent mist whose temperature is controlled.

It is to be noted that a sign in parentheses of each of the aforementioned means indicates correspondence to a specific means described in each of embodiments which will be described later.

### [Advantageous Effects of Invention]

According to the aforementioned configuration, the pass box for decontamination according to the present invention has a pass box main-body including a carrying-in zone, a decontamination zone, an aeration zone, and a carrying-out zone, and a carrying device which carries an article inside the pass box main-body. The carrying-in zone includes an airtightly sealable carrying-in port through which an article is carried in from an outside environment. The decontamination zone includes a decontamination-agent applying device which applies a decontamination agent mist to an outer surface of the article which has been carried by the carrying device from the carrying-in zone in order to decontaminate the outer surface of the carried-in article by a decontamination agent. The aeration zone includes a clean-air applying device which applies a clean air to the surface of the article which has been carried by the carrying device from the decontamination zone in order to remove the decontamination agent remaining on the outer surface of the article after the decontamination. The carrying-out zone carries out the article after the aeration to an inside of a work room in a sterile environment by the carrying device.

Further, in the pass box for decontamination according to the present invention, an amount of a compressed gas supplied from a compressed-gas generating means, an amount of the decontamination agent supplied from a decontamination-agent supply means, and a heating temperature of a heater are controlled, whereby a temperature of a decontamination agent mist applied to the outer surface of the article is controlled. As a result, when the outer surface of a container which accommodates a thermosensitive substance is to be decontaminated, a pass box for decontamination which can promote a complete decontamination effect at a proper temperature and by a decontamination operation in a short time without giving an influence such as a thermal quality change to the thermosensitive substance can be provided.

Moreover, according to the aforementioned configuration, the carrying device operates in a first carrying process, a second carrying process, and a third carrying process. In the first carrying process, a part of an article which has been carried into the carrying-in zone and has not been decontaminated is supported and is carried from the carrying-in zone to the decontamination zone. During this period, a most part other than the part supported in the decontamination zone is decontaminated. In the second carrying process, in a state in which a part of the part decontaminated in the first carrying process is switched to be supported, it is carried from the decontamination zone to the aeration zone. During this period, a part other than the part decontaminated in the first carrying process in the decontamination zone is decontaminated. Further, a most part other than the part supported in the aeration zone is aerated. In the third carrying process, in a state in which a part of the part aerated in the second carrying process is switched to be supported, it is carried from the aeration zone to the carrying-out zone. During this period, a part other than the part aerated in the second carrying process is aerated. As a result, the aforementioned working effect can be exerted more specifically and effectively.

Moreover, according to the aforementioned configuration, it may be so configured that the carrying device is constituted by three units of carrying devices, and a first carrying device operates in the first carrying process, a second carrying device operates in the second carrying process, and a third carrying device operates in the third carrying process. Alternatively, it may be so configured that the carrying device is constituted by two units of carrying devices, and the first carrying device operates in the first carrying process and the third carrying process, and the second carrying device operates in the second carrying process. By means of the above, the aforementioned working effect can be exerted more specifically and effectively.

Moreover, according to the aforementioned configuration, it is preferable that, in the carrying device, a total of each operation time of the first carrying process, the second carrying process, and the third carrying process is within 10 minutes by continuous or semi-continuous operation. Moreover, a temperature of the decontamination agent mist is preferably controlled in a range from 30 to 60°C. By means of the above, the aforementioned working effect can be exerted more specifically and more effectively.

Moreover, according to the aforementioned configuration, the pass box for decontamination according to the present invention includes a first exhaust device and a second exhaust device. The first exhaust device exhausts an air mainly in the carrying-in zone and the decontamination zone. The second exhaust device exhausts the air mainly in the aeration zone and the carrying-out zone. Further, an exhaust amount of the first exhaust device is set larger than the exhaust amount of the second exhaust device, whereby the air in the carrying-in zone and the decontamination zone does not mix in the aeration zone and the carrying-out zone. Moreover, an air pressure of the pass box main-body is set more negative than the air pressure inside the work room, whereby the air in the pass box main-body is not mixed inside the work room.

Moreover, according to the aforementioned configuration, a heater including an introduction port which introduces the mixed gas-liquid, a cylindrical outer cylinder tube, a heat generating body incorporated inside the outer cylinder tube, in parallel to the longitudinal direction of the outer cylinder tube, and an ejection port through which the decontamination agent mist is ejected may be used. In this heater, the mixed gas-liquid is heated while passing between the outer cylinder tube and the heat generating body, and the whole or a part of the mixed gas-liquid becomes the decontamination agent gas. Further, the decontamination agent gas is made into a mist by the compressed gas whose amount is controlled and is changed to the decontamination agent mist whose temperature is controlled. As a result, the aforementioned working effect can be exerted more specifically and more effectively.

Moreover, according to the aforementioned configuration, the heater including the introduction port which introduces the mixed gas-liquid, the cylindrical outer cylinder tube, the heat generating body incorporated inside the outer cylinder tube, in parallel to the longitudinal direction of the outer cylinder tube, and the ejection port through which the decontamination agent mist is ejected, and the heat generating body including a rod-shaped heater disposed in the longitudinal direction thereof and an evaporation tube wound in a spiral state in the longitudinal direction along an outer periphery of the heater may be used. In this heater, the mixed gas-liquid is heated while passing through the inside of the evaporation tube, and the whole or a part of the mixed gas-liquid becomes the decontamination agent gas. Further, the decontamination agent gas is made into a mist by the compressed gas whose amount is controlled and is changed to the decontamination agent mist whose temperature is controlled. As a result, the aforementioned working effect can be exerted more specifically and more effectively.

Moreover, according to the aforementioned configuration, the heater may include a mixed-gas regulator which mixes the decontamination agent gas and the compressed gas between the heat generating body and the ejection port. In this heater, the amount of the compressed gas to be introduced into the mixed-gas regulator is controlled, whereby the decontamination agent gas is made into a mist by the compressed gas and is changed to the decontamination agent mist whose temperature is controlled. As a result, the aforementioned working effect can be exerted more specifically and more effectively.

### BRIEF DESCRIPTION OF DRAWINGS

FIG. 1 is a front view of an isolator device including a pass box for decontamination according to this Embodiment.
FIG. 2 is an internal sectional diagram illustrating a state in which a vial is carried into the pass box for decontamination from an outside environment.
FIG. 3 is an internal sectional diagram illustrating a switching operation of a holding of the vial to a first carrying device.
FIG. 4 is an internal sectional diagram illustrating a state in which the vial is carried by the first carrying device.
FIG. 5 is an internal sectional diagram illustrating a state in which the vial carried by the first carrying device is carried from a carrying-in zone to a decontamination zone.
FIG. 6 is an internal sectional diagram illustrating a state in which the vial carried by the first carrying device is switched to be held by the second carrying device.
FIG. 7 is an internal sectional diagram illustrating a state in which the vial sandwiched by the second carrying device is carried from the decontamination zone to an aeration zone.
FIG. 8 is an internal sectional diagram illustrating a state in which, in the aeration zone, the vial sandwiched by the second carrying device is switched to be held by the first carrying device.
FIG. 9 is an internal sectional diagram illustrating a state in which the vial carried by the first carrying device is carried from the aeration zone to a carrying-out zone.
FIGS. 10 are schematic views illustrating a state in which the vial is carried by a carrier of the first carrying device, in which Fig. 10A illustrates a state before carrying, and Fig. 10B for a carried state.
FIG. 11A is a perspective view illustrating a relationship between the vial to be decontaminated and a mist cover provided inside the decontamination zone, and Fig. 11B is a plan view.
FIGS. 12 are schematic views illustrating a state in which the vial is sandwiched by a sandwiching tool of the second carrying device, in which Fig. 12A illustrates a state before sandwiching, and Fig. 12B for a sandwiched state.
FIG. 13 is a schematic diagram illustrating an example of a configuration of a decontamination-agent applying device.
FIG. 14 is a schematic diagram illustrating another example of a configuration of the decontamination-agent applying device.
FIG. 15 is a sectional view illustrating an example of a heater provided in the decontamination-agent applying device.
FIG. 16 is a sectional view illustrating another example of the heater provided in the decontamination-agent applying device.

### DESCRIPTION OF EMBODIMENTS

Hereinafter, a pass box for decontamination according to the present invention will be explained in detail with reference to Embodiments. It is to be noted that the present invention is not limited only to the following Embodiments.

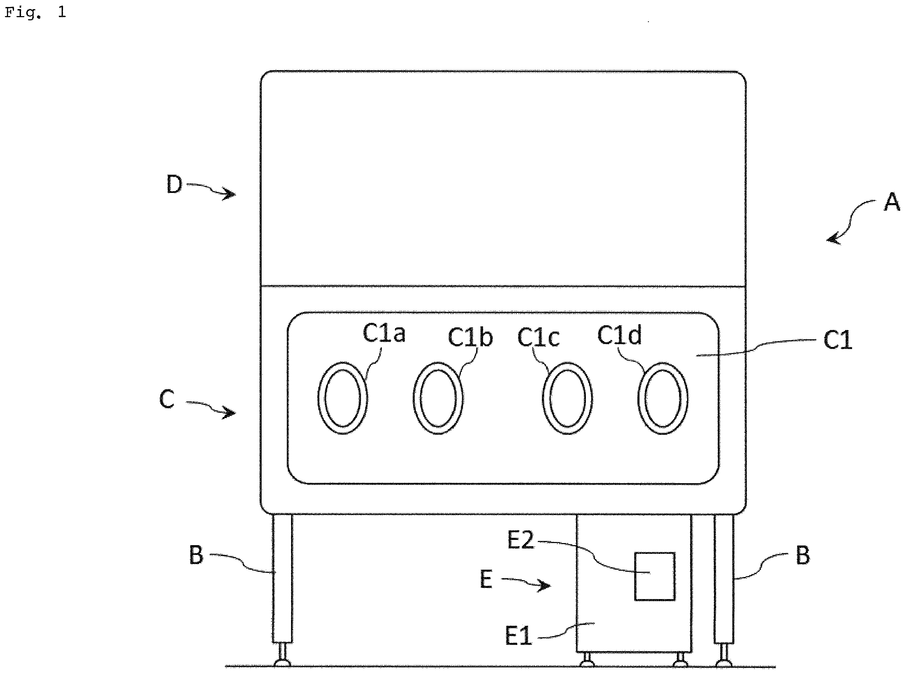

Fig. 1 is a front view of an isolator device including a pass box for decontamination according to this Embodiment. In Fig. 1, the isolator device A is constituted by a chamber C placed on a floor surface by a leg part B and a device chamber D mounted on the chamber C. Moreover, the pass box for decontamination according to this Embodiment has a pass box main-body E and a carrying device (which will be described later) which carries an article in an inside of the pass box main-body E. The pass box main-body E is placed on the floor surface in a state in which an upper-surface wall part thereof is joined to a bottom-surface wall part of the chamber C. On a front-surface wall part E1 of this pass box main-body E, a carrying-in door E2 through which an article is carried into the chamber C is provided (details of which will be described later). It is to be noted that, in this Embodiment, the "article" refers to a vial accommodating a thermosensitive substance (hereinafter, referred to as a "vial").

The chamber C is constituted by a box body made of a stainless, and a work is performed in a sterile environment therein. The chamber C is airtightly shielded from an outside environment where a worker is present. Moreover, on the front-surface wall part of the chamber C, a transparent glass window C1 through which an inside can be visually recognized is provided. In this glass window C1, four openings C1a to C1d for a work which cause the outside and an inside of the chamber C to communicate with each other are opened. At these openings C1a to C1d for work, a glove made of a resin (not shown) is airtightly attached, respectively.

Moreover, on the bottom-surface wall part inside the chamber C, an opening (not shown) which communicates with the upper-surface wall part of the pass box main-body E is provided and serves as a carrying-in port (carrying-out port of the pass box main-body E) through which the vial decontaminated inside the pass box main-body E is carried into the chamber C. It is to be noted that the carrying-in of the vial into the chamber C is performed by the worker in the outside environment through the glove made of a resin (details of which will be described later) .

A periphery of the device chamber D is covered with a wall material made of a metal plate made of a stainless, and devices such as electric / mechanical devices (not shown) are accommodated inside thereof. These devices include a sucking blower including a sucking filter unit which controls an internal environment of the chamber C, an exhaust blower including an exhaust filter unit and the like. Moreover, on the front-surface wall part of this device chamber D, a control panel (not shown) is incorporated.

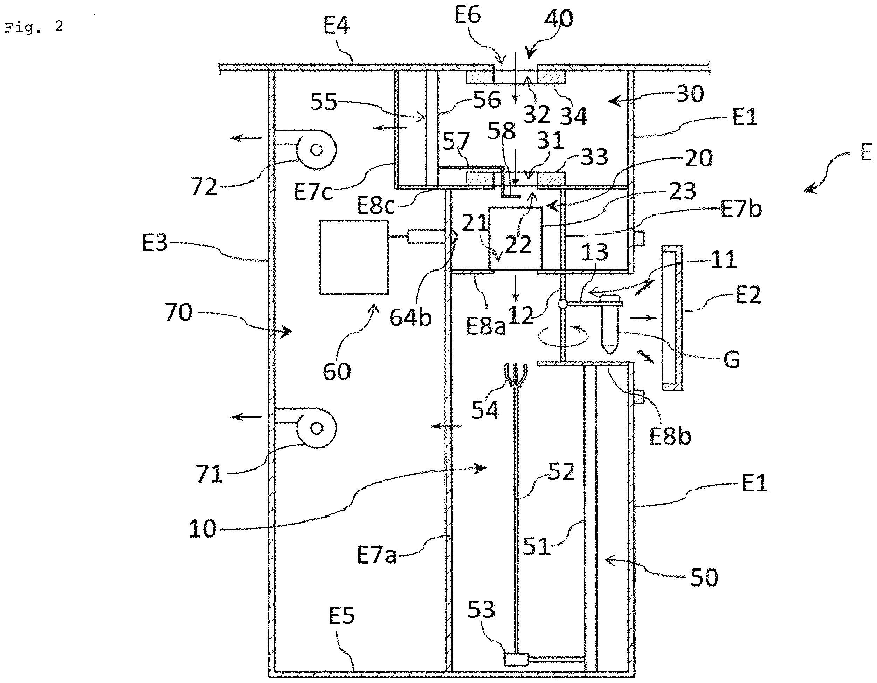

Subsequently, a configuration and an operation of the pass box for decontamination according to this Embodiment will be explained. Fig. 2 is an internal sectional diagram illustrating a state in which a vial is carried into the pass box for decontamination from the outside environment.

In Fig. 2, the pass box main-body E is a box body made of a stainless surrounded by the front-surface wall part E1, a rear-surface wall part E3, an upper-surface wall part E4, a bottom-surface wall part E5, a right-side wall part (depth in the figure), and a left-side wall part (front in the figure, not shown). In the front-surface wall part E1, the carrying-in door E2 through which the vial is carried in is provided. It is to be noted that, in Fig. 2, the carrying-in door E2 is in an open state for carrying in the vial. Moreover, the chamber C of the isolator device A (not shown) is in a state mounted on an upper part of the pass box main-body E. It is to be noted that the upper-surface wall part E4 of the pass box main-body E has a structure also serving the bottom-surface wall part of the chamber C, and a carrying-out port E6 through which the vial is carried out into the chamber C from the inside of the pass box main-body E is opened.

Inside the pass box main-body E, a carrying-in zone 10 where the vial is carried in from the outside environment, a decontamination zone 20 where an outer surface of the carried-in vial is decontaminated by the decontamination agent, an aeration zone 30 where the decontamination agent remaining on the outer surface of the vial after the decontamination is removed, and a carrying-out zone 40 where the vial after the aeration is carried out into the chamber C are provided.

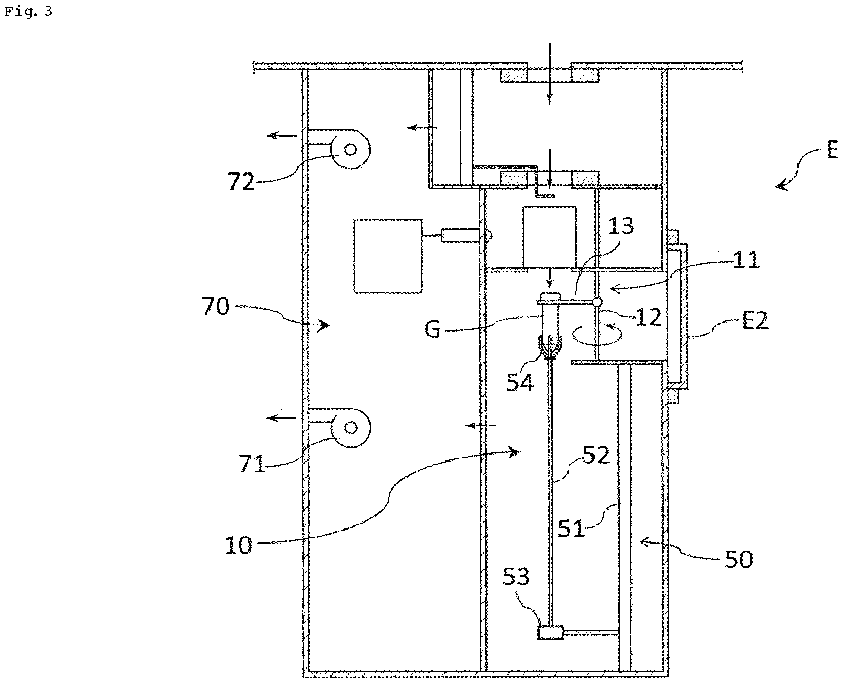

In Fig. 2, the carrying-in zone 10 is a part surrounded by the front-surface wall part E1, the carrying-in door E2, the bottom-surface wall part E5, a vertical wall part E7a, and a lateral wall part E8a. In the carrying-in zone 10, a stand 11 which sandwiches an upper-part side surface of the vial G which has been carried in from the outside environment via the carrying-in door E2 and moves it into the inside is installed. The stand 11 is made of a support shaft 12 and a sandwiching shaft 13, and the sandwiching shaft 13 which sandwiches the vial G rotates around the support shaft 12 and transfers the vial G into the carrying-in zone 10 (see Fig. 2 and Fig. 3).

Moreover, inside the carrying-in zone 10, a first carrying device 50 which receives the vial G from the stand 11 and carries it to the carrying-out zone 40 via the decontamination zone 20 and the aeration zone 30 is provided. The first carrying device 50 has an elevation shaft 51 fixed between the bottom-surface wall part E5 and a lateral wall part E8b, a carrying shaft 52 which moves in an up-down direction along the elevation shaft 51, and a rotation mechanism 53 which rotates the carrying shaft 52. Moreover, on an upper end part of the carrying shaft 52, a carrying tool 54 which carries a bottom part of the vial G is provided. It is to be noted that an operation of the first carrying device 50 will be described later.

In Fig. 2, the decontamination zone 20 is a part surrounded by the vertical wall part E7a, a vertical wall part E7b, the lateral wall part E8a, and a lateral wall part E8c. In the decontamination zone 20, an opening 21 is opened at a center part of the lateral wall part E8a and an opening 22 at a center part of the lateral wall part E8c. These openings 21, 22 are parts through which the vial G carried by the first carrying device 50 passes (details will be described later). Moreover, in the vertical wall part E7a of the decontamination zone 20, an ejection port 64b of a decontamination-agent applying device 60, through which the decontamination agent is ejected (in this Embodiment, the decontamination agent mist) is installed (details will be described later). Moreover, inside the decontamination zone 20, a mist cover 23 which surrounds the vial G and efficiently promotes the application of the decontamination agent mist is provided (details will be described later).

In Fig. 2, the aeration zone 30 is a part surrounded by the front-surface wall part E1, a vertical wall part E7c, the lateral wall part E8c, and the upper-surface wall part E4. In the aeration zone 30, an opening 31 (in common with the opening 22 of the decontamination zone) is opened at a center part of the lateral wall part E8c, and an opening 32 (in common with the carrying-out port E6) at the center part of the upper-surface wall part E4. These openings 31, 32 are parts through which the vial G carried by the first carrying device 50 or sandwiched by a second carrying device 55, which will be described later, passes. Moreover, at the openings 31, 32, ring-shaped clean-air applying devices 33, 34 which blow a clean air for the aeration toward the vial G, respectively, are provided along each of the openings 31, 32. It is to be noted that the opening 32 corresponds to the carrying-out port E6 through which the vial is carried out from the inside of the aforementioned pass box main-body E into the chamber C.

Moreover, inside the aeration zone 30, the second carrying device 55 which receives the vial G from the first carrying device 50 and carries it from the decontamination zone 20 to the aeration zone 30 is provided. The second carrying device 55 has an elevation shaft 56 fixed between the lateral wall part E8c and the upper-surface wall part E4 and a sandwiching shaft 57 which moves in the up-down direction along the elevation shaft 56. Moreover, at a distal end part of the sandwiching shaft 57, a sandwiching tool 58 which sandwiches an upper-part side surface of the vial G is provided. It is to be noted that an operation of the second carrying device 50 will be described later.

In Fig. 2, the carrying-out zone 40 is a part in common with an upper part of the aeration zone 30. That is, it is the carrying-out port E6 part which has a function of carrying out the vial from the inside of the pass box main-body E into the chamber C. It is to be noted that, in this Embodiment, the carrying-out port E6 corresponds to the opening 32 of the aeration zone 30, and the carrying-out zone 40 and the upper part of the aeration zone 30 are in common. It is to be noted that this is not limiting, and the carrying-out zone 40 may be provided separately from the aeration zone 30.

In Fig. 2, a part surrounded by the rear-surface wall part E3, the upper-surface wall part E4, the bottom-surface wall part E5, the vertical wall part E7a, the vertical wall part E7c, and a part of the lateral wall part E8c is a machine room 70, and the aforementioned decontamination-agent applying device 60 and two units of exhaust blowers 71, 72 are provided. Moreover, in the machine room 70, an exhaust filter unit, a hydrogen peroxide decomposition unit, and devices related to the drive and the control of the pass box for decontamination are accommodated (none of them is shown).

Here, a flow of the air inside the pass box main-body E will be explained. The two units of the exhaust blowers 71, 72 provided inside the pass box main-body E are operated, whereby the air pressure inside the pass box main-body E is made lower than the air pressure inside the chamber C of the isolator device A. As a result, the air inside the pass box main-body E (including the decontamination agent and the like) is prevented from mixing into the chamber C through the carrying-out port E6. Arrows described in each of the openings and the wall parts in Fig. 2 indicate flow directions of the air.

Further, it is preferable that exhaust amounts of the two units of exhaust blowers 71, 72 are adjusted. The exhaust blower 71 is located on a lower part of the machine room 70 and exhausts mainly the air in the carrying-in zone 10 and the decontamination zone 20. The carrying-in zone 10 is a part to which the vial G before the decontamination is carried in, and a cleanliness degree of the air is low. Moreover, the decontamination zone 20 is a part to which the decontamination agent is applied, and the decontamination agent is mixed in the air.

On the other hand, the exhaust blower 72 is located on an upper part of the machine room 70 and exhausts an air mainly in the aeration zone 30 and the carrying-out zone 40. The aeration zone 30 is a part where the decontamination agent is removed, and the decontamination agent is mixed in the air. Moreover, the carrying-out zone 40 is a part where the vial G after the aeration is carried out, and it is preferable that, though the degree of cleanliness is high, the clean air flows in from the inside of the chamber C. Thus, the exhaust amount of the exhaust blower 71 is made larger than the exhaust amount of the exhaust blower 72, whereby the air in the carrying-in zone 10 and the decontamination zone 20 is not mixed in the aeration zone 30 and the carrying-out zone 40.

In the configuration as above, an operation of the pass box for decontamination will be explained specifically. The pass box for decontamination according to this Embodiment performs a decontamination operation while carrying the vial G in a state supported (carried or sandwiched) by the first carrying device 50 or the second carrying device 55 in the order of the carrying-in zone 10, the decontamination zone 20, the aeration zone 30, and the carrying-out zone 40. Here, the decontamination operation will be explained in the order of the carrying-in process, the first carrying process, the second carrying process, and the third carrying process along the carrying of the vial G with reference to Figs. 2 to 9. It is to be noted that each of the carrying processes can be performed continuously or semi-continuously. In the continuous carrying operation, the decontamination and the aeration are performed while the vial G is moved all the time. On the other hand, in the semi-continuous carrying operation, the movement at the decontamination or the aeration of the vial G is stopped, and the operation time is ensured.

### <Carrying-in Process>

Fig. 2 is an internal sectional diagram illustrating a state in which the vial has been carried into the pass box for decontamination from the outside environment as described above. The worker (not shown) in the outside environment of the isolator device A opens the carrying-in door E2 and causes the vial G which accommodates the thermosensitive substance to be supported on the stand 11 in the carrying-in zone 10. It is to be noted that a method of supporting the vial is not particularly limited but can be selected as appropriate in accordance with a shape of the vial. In this Embodiment, in accordance with the shape of the vial G, an upper-part side surface (below an upper-part cap) is sandwiched by the sandwiching shaft 13.

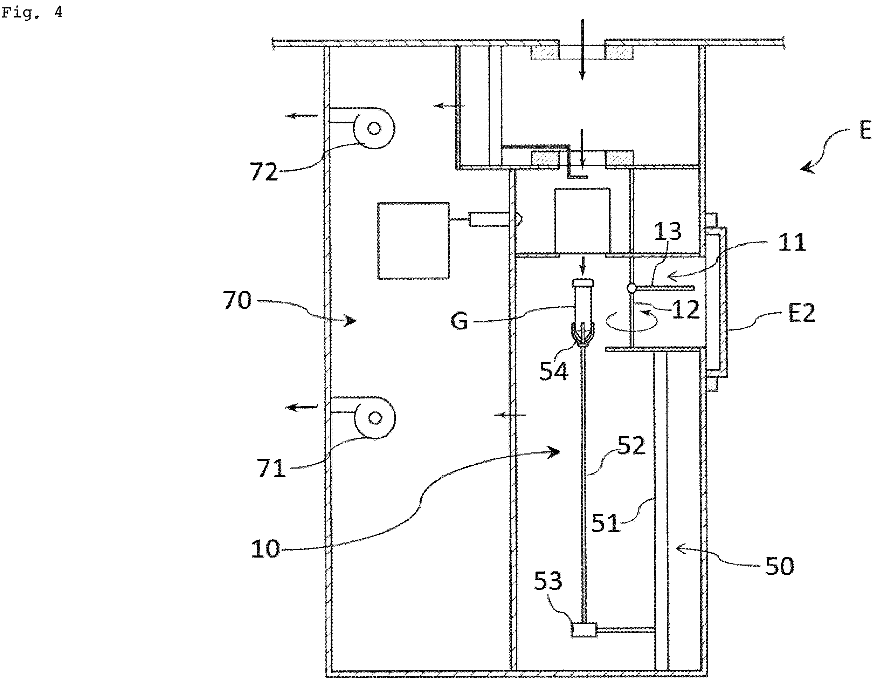

Subsequently, the holding of the vial G is switched from the stand 11 to the first carrying device 50. Fig. 3 is the internal sectional diagram illustrating an operation of the switching the holding of the vial to the first carrying device. It is to be noted that, in Figs. 3 to 9 shown below, only signs of parts for which the carrying process will be explained are described. In Fig. 3, the sandwiching shaft 13 in the state in which the vial G is sandwiched is rotated around the support shaft 12 and moved to an upper part of the carrying tool 54 located on an upper end part of the carrying shaft 52 of the first carrying device 50. Further, the carrying shaft 52 of the first carrying device 50 is moved in an upper direction along the elevation shaft 51, a bottom part of the vial G is supported (carried, here) by the carrying tool 54 on the upper end part of the carrying shaft 52 and the holding thereof is switched. Subsequently, the sandwiching shaft 13 of the stand 11 is rotated around the support shaft 12 and is returned to a direction of the carrying-in door E2 (see Figs. 3 and 4).

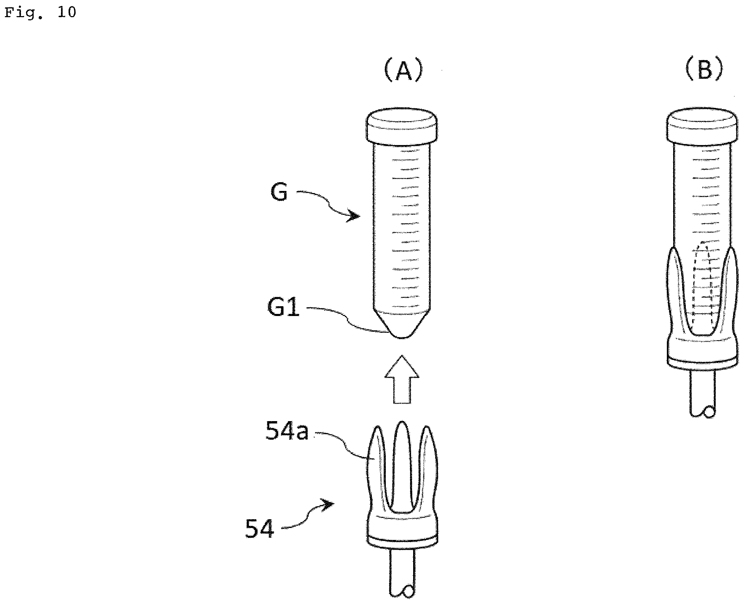

Here, the carrying of the bottom part of the vial G by the first carrying device 50 will be explained. Figs. 10 are schematic views illustrating a state in which the vial is carried by the carrying tool of the first carrying device, in which Fig. 10A illustrates a state before the carrying, while Fig. 10B for a carried state. The vial G shown in Figs. 10 was made of a polyethylene material and had a height of 7 cm, a diameter of 2 cm, and a capacity of 15 ml. The carrying tool 54 of the first carrying device has three pieces of claws 54a open upward so that the bottom part G1 of the vial G can be stably carried. It is to be noted that a method of supporting the vial is not limited to the carrying as above, but other support tools may be selected as appropriate in accordance with a shape of the vial.

### <First Carrying Process>

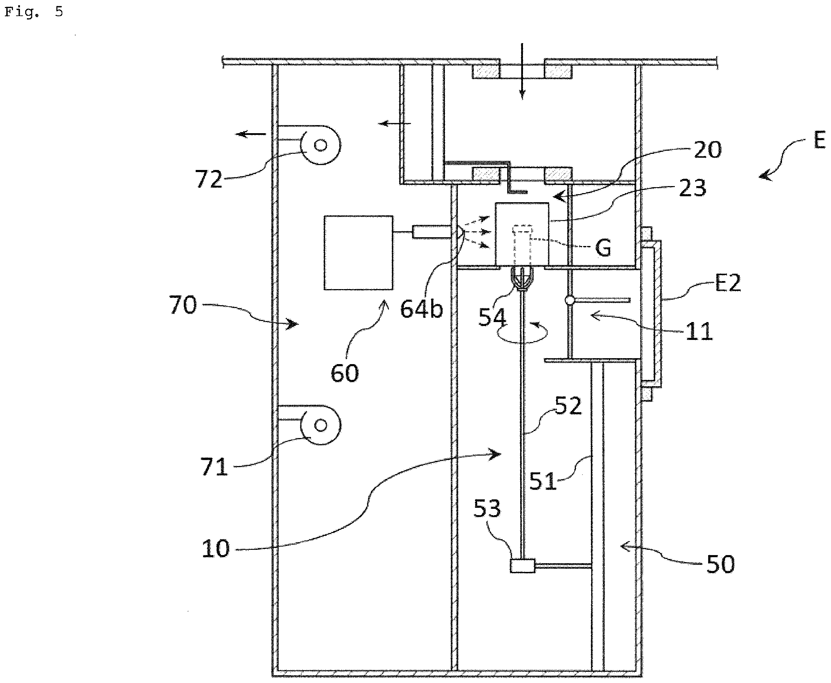

Subsequently, the decontamination operation of the vial G is started. Fig. 4 is an internal sectional diagram illustrating a state in which the vial is carried by the first carrying device. In this state, the preparation for the decontamination operation of the vial G is complete. In the first carrying process, the carrying shaft 52 of the first carrying device 50 is further moved in the upper direction along the elevation shaft 51, and the vial G carried by the carrying tool 54 of the carrying shaft 52 is carried from the carrying-in zone 10 to the decontamination zone 20. Fig. 5 is an internal sectional diagram illustrating a state in which the vial carried by the first carrying device is carried from the carrying-in zone to the decontamination zone. In Fig. 5, the decontamination agent mist is ejected from the ejection port 64b of the decontamination-agent applying device 60. In this first carrying process, in the vial G, a most part other than the part carried by the carrying tool 54 is decontaminated.

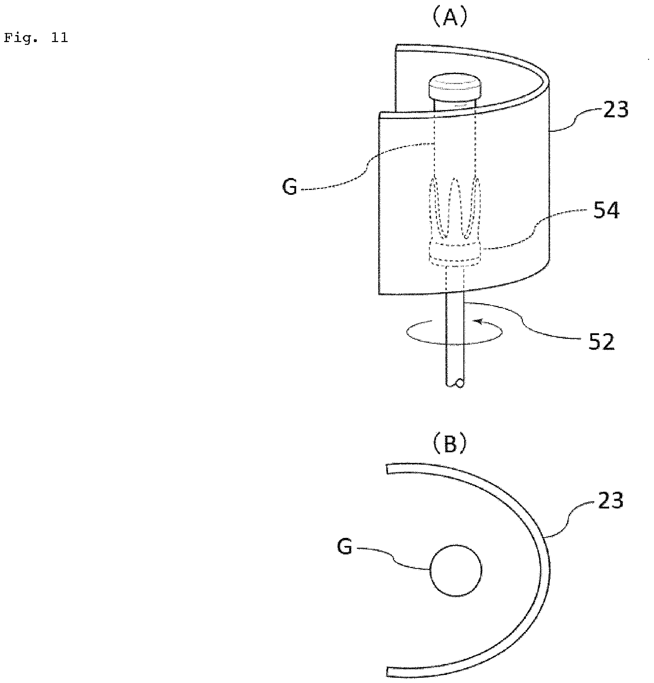

In Figs. 11, Fig. 11A is a perspective view and Fig. 11B is a plan view illustrating a relationship between the vial to be decontaminated and the mist cover provided inside the decontamination zone. The mist cover 23 is a semi-cylindrical wall material and is disposed and encloses the vial G to be decontaminated therein. In Figs. 11, when the vial G carried by the carrying tool 54 of the first carrying device 50 is carried to the decontamination zone 20, the decontamination agent mist is ejected from the decontamination-agent applying device 60 (not shown) installed in the left direction in the illustration. The ejected decontamination agent mist is applied to the front surface of the vial G and goes around to the rear surface of the vial G along an inner surface of the mist cover 23. As a result, the decontamination agent mist is uniformly applied to the entire outer surface of the vial G.

Moreover, in the decontamination zone 20, the carrying shaft 52 is rotated by the rotation mechanism 53 of the first carrying device 50. As a result, the decontamination agent mist can be further uniformly applied to the entire outer surface of the vial G. By means of the above, the decontamination agent mist whose temperature is controlled can be applied to the outer surface of the vial G in an appropriate amount and efficiently and thus, the improvement of the decontamination efficiency and the reduction of the decontamination time can be promoted.

It is to be noted that, in the decontamination operation, it is preferable that the exhaust blower 71 is stopped (see Fig. 5), and the exhaust amount of the exhaust blower 72 is adjusted and operated. The exhaust blower 71 is stopped, whereby the decontamination agent mist supplied in the decontamination zone 20 can be prevented from being exhausted more than necessary. On the other hand, the exhaust amount of the exhaust blower 72 is adjusted, and the clean air flowing in from the inside of the chamber C is maintained.

### <Second Carrying Process>

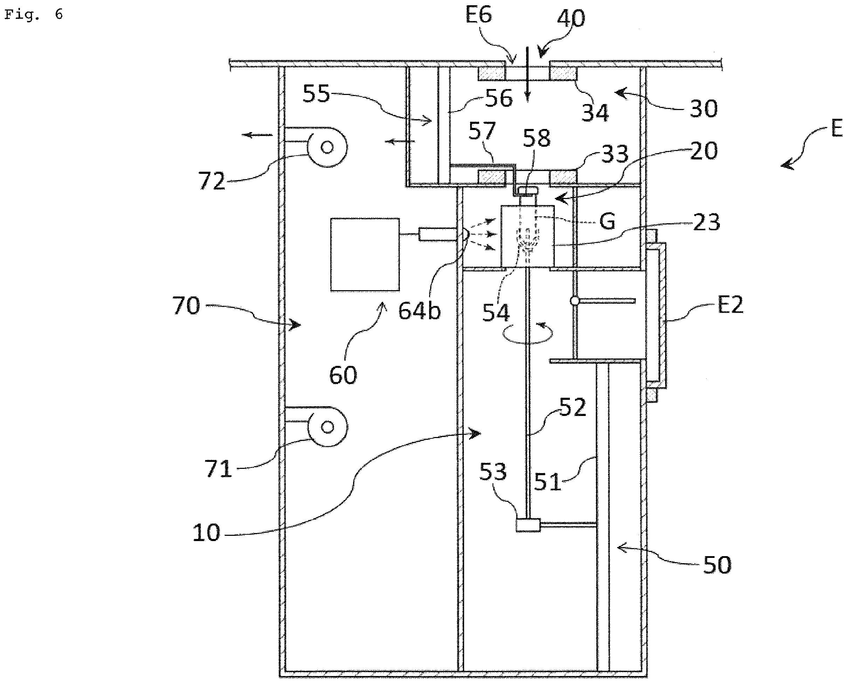

In the second carrying process, the holding of the vial G is switched from the first carrying device 50 to the second carrying device 55. Fig. 6 is an internal sectional diagram illustrating a state in which the holding of the vial carried by the first carrying device is switched to the second carrying device. In this state, too, it is preferable that the exhaust blower 71 is stopped, the exhaust amount of the exhaust blower 72 is adjusted, and the operation is performed.

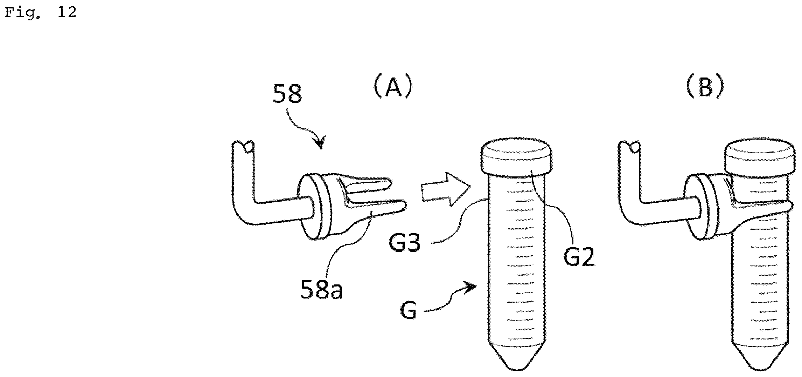

Here, the sandwiching of the upper-part side surface of the vial G by the second carrying device 55 will be explained. Figs. 12 are schematic views illustrating a state in which the vial is sandwiched by the sandwiching tool of the second carrying device, in which Fig. 12A illustrates a state before the sandwiching, while Fig. 12B for a sandwiched state. The vial G shown in Figs. 12 was made of the same polyethylene material as that in Figs. 10 and had a height of 7 cm, a diameter of 2 cm, and a capacity of 15 ml. The sandwiching tool 58 of the second carrying device has two pieces of claws 58a open in a horizontal direction, and an upper-part side surface G3 immediately below a cap G2 of the vial G can be stably sandwiched. It is to be noted that a method of supporting the vial is not limited to the sandwiching as above, but other support tools may be selected as appropriate in accordance with a shape of the vial.

Subsequently, the sandwiching shaft 57 of the second carrying device 55 is moved in the upper direction along the elevation shaft 56, and the vial G carried by the sandwiching tool 58 of the sandwiching shaft 57 is carried from the decontamination zone 20 to the aeration zone 30. It is to be noted that, in the decontamination zone 20, the holding of the vial G is switched from the first carrying device to the second carrying device, whereby the part which could not be decontaminated in the first carrying process (particularly the part carried by the first carrying device) can be decontaminated.

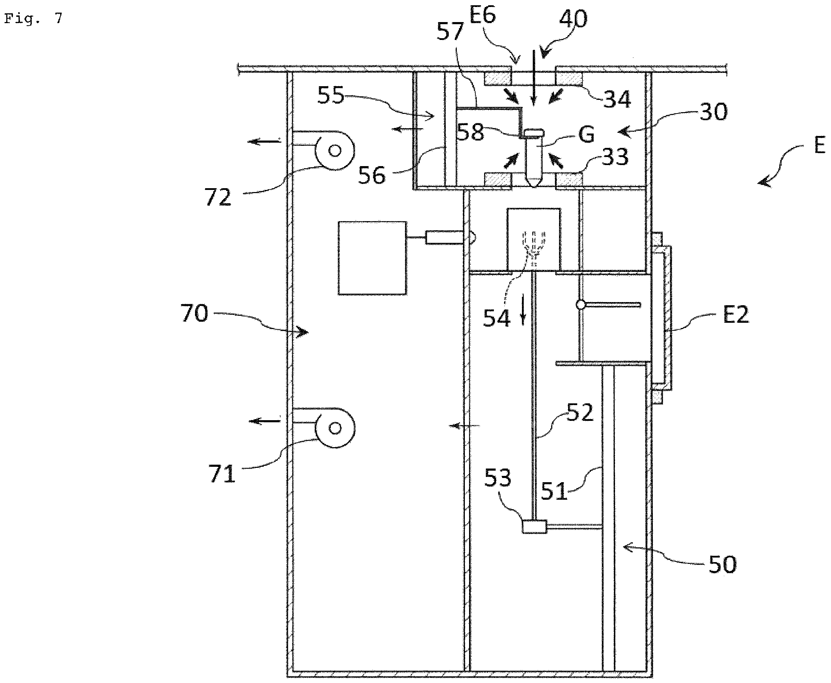

Fig. 7 is an internal sectional diagram illustrating a state in which the vial sandwiched by the second carrying device is carried from the decontamination zone to the aeration zone. In this second carrying process, the most part other than the part sandwiched by the sandwiching shaft 57 of the vial G is aerated, and the decontamination agent remaining on the surface of the vial G is removed. In the aeration, the clean air is blown from the two units of ring-shaped clean-air applying devices 33, 34 provided at the openings 31, 32 toward the surface of the vial G.

It is to be noted that, from the clean-air applying device 33 on a lower part provided at the opening 31, the clean air is blown toward above the center part of the ring. On the other hand, from the clean-air applying device 34 on an upper part provided at the opening 32, the clean air is blown toward below the center part of the ring. As a result, the clean air can be blown to the whole surface of the vial G passing through the centers of the ring shapes of the two units of clean-air applying devices 33, 34.

### <Third Carrying Process>

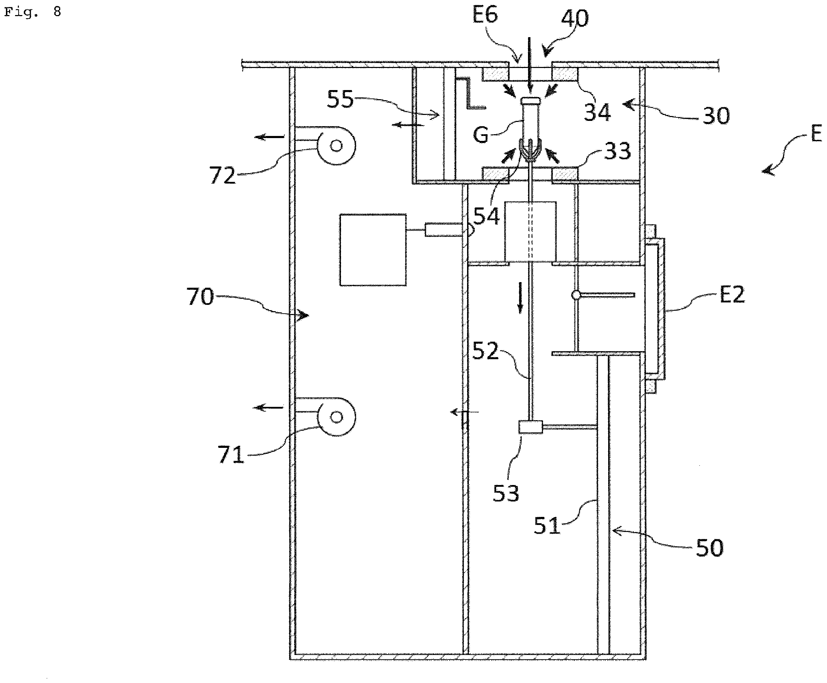

In the third carrying process, the holding of the vial G is switched again from the second carrying device 55 to the first carrying device 50. Fig. 8 is an internal sectional diagram illustrating a state in which the holding of the vial sandwiched by the second carrying device is switched to the first carrying device in the aeration zone. In this state, the exhaust blower 71 and the exhaust blower 72 are both operated.

In this third carrying process, the sandwiching shaft 52 of the first carrying device 50 is moved in the upper direction along the elevation shaft 51, and the vial G carried by the sandwiching tool 54 of the sandwiching shaft 52 is carried from the aeration zone 30 to the carrying-out zone 40. It is to be noted that, in the aeration zone 30, the holding of the vial G is switched from the second carrying device to the first carrying device, whereby the part which could not be aerated in the second carrying process (particularly the part sandwiched by the second carrying device) can be aerated.

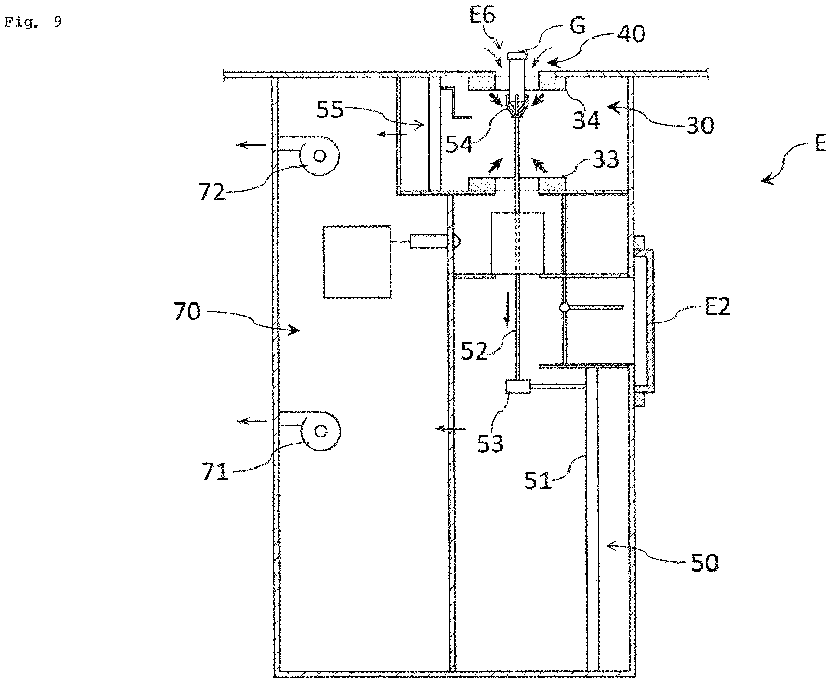

Fig. 9 is an internal sectional diagram illustrating a state in which the vial carried by the first carrying device is carried from the aeration zone to the carrying-out zone. The upper part (cap part) of the vial G is in a state of being carried out from the carrying-out port E6 (corresponding to the opening 32) into the chamber C of the isolator device A. The worker in the outside environment carries the vial G into the chamber C by using the glove made of a resin of the chamber C. It is to be noted, as described above, the clean air flows from the carrying-out port E6 from the inside of the chamber C. Therefore, it is not necessarily required to provide an opening/closing door at the carrying-out port E6. However, it may be so configured that the opening/closing door is provided and is opened only when the vial G is carried in.

Subsequently, the decontamination-agent applying device 60 in this Embodiment will be explained. The decontamination-agent applying device in this Embodiment ejects the decontamination agent mist, which is controlled to a temperature and does not influence the thermosensitive substance, accommodated inside the vial. It is to be noted that, in this Embodiment, a hydrogen peroxide solution (H₂O₂ solution) was used as the decontamination agent. It is to be noted that the decontamination agent is not limited to the hydrogen peroxide solution, but any liquid-state decontamination agent may be used.

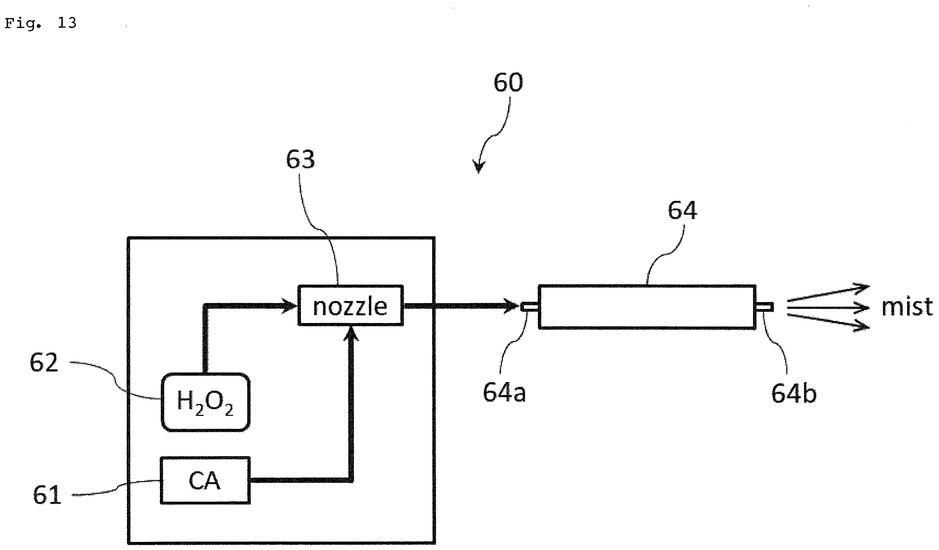

Fig. 13 is a schematic diagram illustrating an example of a configuration of the decontamination-agent applying device. In Fig. 13, the decontamination-agent applying device 60 is constituted by a compressed-gas generating device 61, a decontamination-liquid supplying device 62, a mixed gas-liquid regulator 63, and a heater 64.

In this Embodiment, a compressor which generates a compressed air is employed as the compressed-gas generating device 61, and a supply amount of the compressed air is controlled. The decontamination-liquid supply device 62 is constituted by a hydrogen-peroxide solution tank, a piping, a pump, and a load cell (none of them is shown), and a supply amount of the hydrogen peroxide solution is controlled. Here, a concentration of the hydrogen peroxide solution stored in the hydrogen-peroxide solution tank is not particularly limited, but in general, a handling of hazardous objects is considered, and those with 30 to 35 % by weight is preferably used.

The mixed gas-liquid regulator 63 mixes the hydrogen peroxide solution supplied from the decontamination-liquid supply device 62 and the compressed air supplied from the compressed-gas generating device 61, makes them into a mist, and generates a mixed gas-liquid (hereinafter, referred to as a "primary mist") . It is to be noted that, in this Embodiment, a two-fluid spray nozzle was employed as the mixed gas-liquid regulator 63. It is to be noted that, in this Embodiment, the two-fluid spray nozzle is used as the mixed gas-liquid regulator, but this is not limiting, and other spray nozzles or mist generators by an ultrasonic wave such as an ultrasonic humidifier, a nebulizer, and a piezo-atomizer may be used.

The heater 64 efficiently heats the primary mist supplied from the mixed gas-liquid regulator 63. Inside the heater 64, the whole or a part of the primary mist is vaporized, and a hydrogen peroxide gas and a steam are generated. The hydrogen peroxide gas and the steam generated inside the heater 64 are cooled by the compressed air mixed in the primary mist, made into a mist again, become a secondary mist, and become the decontamination agent mist to be ejected from the heater 64. In Fig. 13, one end part 64a of the heater 64 is connected to a discharger of the mixed gas-liquid regulator 63, and the primary mist is supplied to the heater 64. Moreover, the other end part 64b of the heater 64 becomes the ejection port 64b of the decontamination-agent applying device 60 and supplies the decontamination agent mist to the vial G.

In this Embodiment, the amount of the compressed air supplied from the compressed-gas generating device 61, the amount of the hydrogen peroxide solution supplied from the decontamination-liquid supply device 62, and a heating temperature of the heater 64 are controlled, whereby the temperature of the decontamination agent mist applied from the ejection port 64b of the decontamination-agent applying device 60 to the outer surface of the vial G can be controlled. That is, the supply amount of the compressed air supplied from the compressed-gas generating device 61 and the heating temperature of the heater 64 are controlled, and the decontamination agent gas (the hydrogen peroxide gas and the steam) generated inside the heater 64 is changed to the decontamination agent mist (secondary mist) whose temperature is controlled.

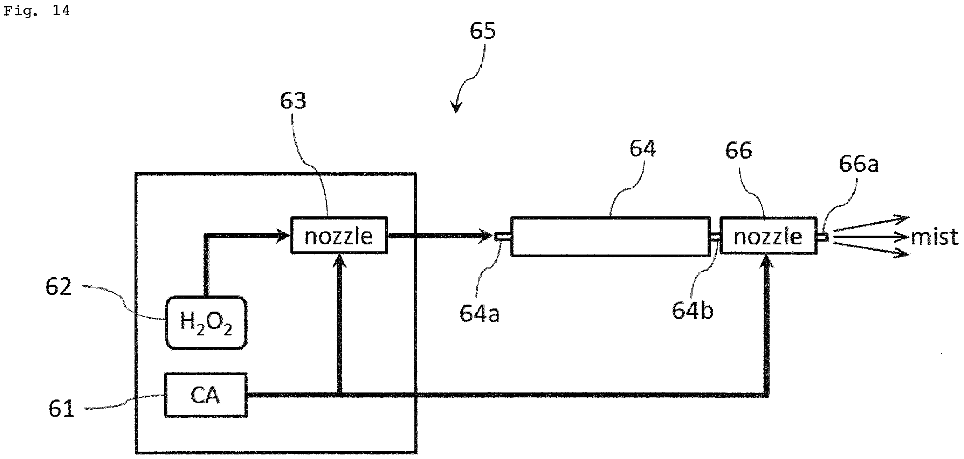

Here, another embodiment of the decontamination-agent applying device will be explained. Fig. 14 is a schematic diagram illustrating another example of the configuration of the decontamination-agent applying device. In Fig. 14, a decontamination-agent applying device 65 is constituted by the compressed-gas generating device 61, the decontamination-liquid supply device 62, the mixed gas-liquid regulator 63, the heater 64, and a mixed-gas regulator 66. In the decontamination-agent applying device 65, to the same configuration as the decontamination-agent applying device 60 in Fig. 13, the mixed-gas regulator 66 is newly added.

The mixed-gas regulator 66 is connected to the end part 64b of the heater 64, and a compressed air is supplied thereto from the compressed-gas generating device 61. The supply amount of the compressed air supplied from the compressed-gas generating device 61 inside the mixed-gas regulator 66 is controlled, whereby the decontamination agent gas (the hydrogen peroxide gas and the steam) generated inside the heater 64 is changed to the decontamination agent mist (secondary mist) whose temperature is controlled. In Fig. 14, the one end part 66a of the mixed-gas regulator 66 becomes an ejection port 66a of the decontamination-agent applying device 65 and supplies the decontamination-agent mist to the vial G.

Here, the mist will be explained. The primary mist according to this Embodiment should be interpreted in a wider sense and is assumed to include a state of droplets of the decontamination agent which was refined and is floating in the air, a state in which the gas and the droplets of the decontamination agent are mixed, a state in which the decontamination agent repeats a phase change of condensation and evaporation between the gas and the droplets and the like. Moreover, a grain diameter is also interpreted in a wider sense including a mist, a fog, a droplet and the like finely classified depending on the cases.

On the other hand, the secondary mist according to this Embodiment is considered to be a mist more refined than the primary mist. The decontamination agent gas (the hydrogen peroxide gas and the steam) generated inside the heater is made into a mist again, and it is considered to be mainly a mist (defined to be at equal to or smaller than 10 µm in some cases) or a fog (defined to be at equal to or smaller than 5 µm in some cases) in terms of a grain diameter. As a result, it is considered that the decontamination efficiency is improved as the temperature is controlled.

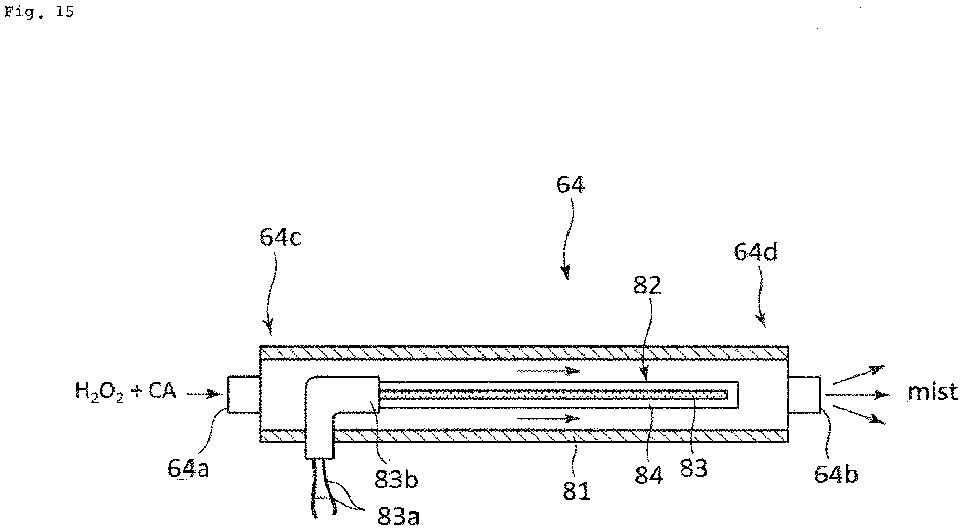

Here, a structure of the heater 64 employed in this Embodiment will be explained. Fig. 15 is a sectional diagram illustrating an example of a heater provided in the decontamination-agent applying device. In Fig. 15, the heater 64 is constituted by a cylindrical outer cylinder tube 81 extending from one end part 64c to the other end part 64d and a heat generating body 82 incorporated therein, in parallel to the longitudinal direction of the outer cylinder tube 81. The outer cylinder tube 81 is formed of a cylinder made of a stainless. The heat generating body 82 includes a heater 83 extending in parallel to the longitudinal direction of the outer cylinder tube 81. It is to be noted that, a gap through which the mixed gas-liquid (primary mist) passes is provided between an inner peripheral surface of the outer cylinder tube 81 and the heat generating body 82.

The heater 83 is installed on an outer-peripheral end part (on a side of the one end part 64c of the heater 64) of the outer cylinder tube 81 by a connection terminal 83b made of a silicon rubber and generates a heat upon receipt of the supply of the electricity from an electric wire 83a. It is to be noted that the heater 83 whose temperature would become high has a surface thereof covered with a quartz glass 84. Moreover, in this Embodiment, an inner peripheral surface of the outer cylinder tube 81 is also covered with the quartz glass. It is to be noted that a structure of the heater 83 is not particularly limited but it may be a rod-shaped heater or a coil-shaped heater. Moreover, the covering with the quartz glass on the surface of the heater or the inner peripheral surface of the outer cylinder tube is not necessarily required, but in this Embodiment, the prevention of a dust generation and the improvement of the thermal efficiency are promoted.

In the heater 64 as above, the atomized mixed gas-liquid (primary mist) supplied from the mixed gas-liquid regulator 63 is introduced into the heater 64 from the introduction port 64a opened in one end part 64c of the heater 64. The primary mist having been introduced into the heater 64 passes through the gap between the outer cylinder tube 81 and the heat generating body 82, while being heated by the heat generating body 82, and moves toward the ejection port 64b opened in the other end part 64d of the heater 64. During this period, the hydrogen peroxide solution in the mixed gas-liquid is heated by the heat generating body 82 and is evaporated (evaporated mainly on the surface of the heat generating body 82) and becomes the decontamination agent gas (the hydrogen peroxide gas and the steam). The supply amount of the compressed air supplied from the compressed-gas generating device 61 is controlled, and this decontamination agent gas is cooled and changed to the decontamination agent mist (secondary mist) whose temperature is controlled. This decontamination agent mist is ejected from the ejection port 64b. It is to be noted that the temperature of the decontamination agent mist to be ejected may be controlled while the temperature of the decontamination agent mist at the ejection port 65b is measured by a temperature sensor.

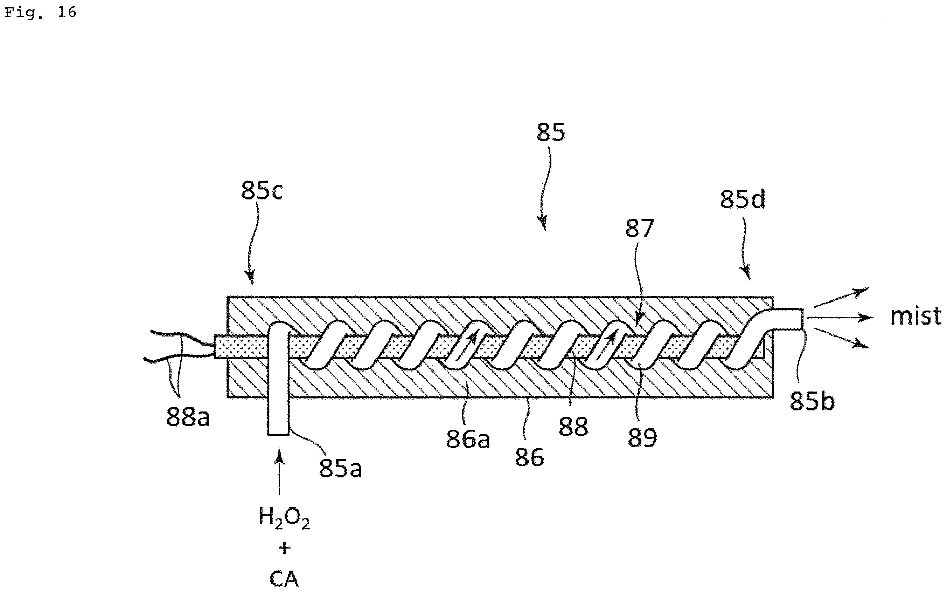

Here, another Embodiment of the heater will be explained. Fig. 16 is a sectional diagram illustrating another example of the heater provided in the decontamination-agent applying device. In Fig. 16, a heater 85 is constituted by a cylindrical outer cylinder tube 86 extending from one end part 85c to the other end part 85d and a heat generating body 87 incorporated therein, in parallel to the longitudinal direction of the outer cylinder tube 86. The outer cylinder tube 86 is formed of a cylinder made of a stainless, and an insulating material 86a is filled therein. The heat generating body 87 includes a rod-shaped cartridge heater 88 extending in parallel to the longitudinal direction of the outer cylinder tube 86 and an evaporation tube 89 wound around in a spiral state in the longitudinal direction along an outer periphery of the cartridge heater 88. It is to be noted that the cartridge heater 88 and the evaporation tube 89 are covered with the insulating material 86a. The cartridge heater 88 is installed on one end part (on a side of the one end part 85c of the heater 85) of the outer cylinder tube 86 and generates a heat upon receipt of the supply of the electricity from an electric wire 88a.

In the heater 85 as above, the atomized mixed gas-liquid (primary mist) supplied from the mixed gas-liquid regulator 63 is introduced into the evaporation tube 89 from the introduction port 85a of the evaporation tube 89 opened in the outer periphery of the one end part 85c of the heater 85. The primary mist introduced into the evaporation tube 89 passes through the inside of the evaporation tube 89 while being heated by the cartridge heater 88 in contact with the evaporation tube 89 and moves toward the ejection port 85b of the evaporation tube 89 opened in the other end part 85d of the heater 85. During this period, the hydrogen peroxide solution in the mixed gas-liquid is heated by the cartridge heater 88 and evaporated and becomes the decontamination agent gas (the hydrogen peroxide gas and the steam). The supply amount of the compressed air supplied from the compressed-gas generating device 61 is controlled, and this decontamination agent gas is cooled and changed to the decontamination agent mist (secondary mist) whose temperature is controlled. This decontamination agent mist is ejected from the ejection port 85b. It is to be noted that the temperature of the decontamination agent mist to be ejected may be controlled while the temperature of the decontamination agent mist at the ejection port 85b is measured by a temperature sensor.

As explained above, according to this Embodiment, when the outer surface of the container which accommodates the thermosensitive substance is to be decontaminated, a pass box for decontamination which can achieve a complete decontamination effect at a proper temperature and by the decontamination operation in a short time without giving an influence such as a thermal quality change to the thermosensitive substance can be provided.

It is to be noted that, in working of the present invention, not limited to the aforementioned embodiment, various modifications as below can be cited.
(1) In the aforementioned Embodiment, the holding of the vial carried in in the carrying-in process is switched from the stand to the first carrying device. However, this is not limiting, but the stand does not have to be employed. In this case, when the worker caries the vial into the pass box for decontamination in the carrying-in process, the vial may be caused to be carried directly by the carrying tool of the first carrying device.
(2) In the aforementioned Embodiment, the first carrying device or the second carrying device elevates the carrying shaft or the sandwiching shaft in the up-down direction along the elevation shaft. However, this is not limiting, and the elevation mechanism and other operation mechanisms may be selected as appropriate by considering the shape or the size of the container such as a vial. Moreover, a small-sized robot arm and the like may be employed.
(3) In the aforementioned Embodiment, in the first carrying process to the third carrying process, the container such as a vial is carried by two units of the carrying devices, that is, the first carrying device and the second carrying device. However, this is not limiting, and in the first carrying process to the third carrying process, the container such as a vial may be carried separately by three units of the carrying devices.
(4) In the aforementioned Embodiment, in the first carrying process to the third carrying process, the container such as a vial is carried in the up-down direction. However, this is not limiting, and it may be so configured that a carrying direction of each of the carrying devices is set to a horizontal direction, and the container such as a vial is carried laterally. In that case, the pass box for decontamination may be disposed on the side wall part of the isolator device.
(5) In the aforementioned embodiment, the first carrying device or the second carrying device carries or sandwiches the container such as a vial in the carrying. However, this is not limiting, and it may be so configured that the container such as a vial is placed on a mesh-state stand and carried in a state where the container and the stand are in point-contact. Further, the carrying may be performed while the contact point between the container and the stand is shifted and the mesh-state stand is vibrated.

### REFERENCE SIGNS LIST

10 Carrying-in zone
11 Stand
12 Support shaft
13 Sandwiching shaft
20 Decontamination zone
21, 22 Opening
30 Aeration zone
31, 32 Opening
33, 34 Clean-air applying device
40 Carrying-out zone
50 First carrying device
51 Elevation shaft
52 Carrying shaft
53 Rotation mechanism
54 Carrying tool
55 Second carrying device
56 Elevation shaft
57 Sandwiching shaft
58 Sandwiching tool
60, 65 Decontamination-agent applying device
61 Compressed-gas generating device
62 Decontamination-liquid supply device
63 Mixed gas-liquid regulator
64, 85 Heater
64b Ejection port
66 Mixed-gas regulator
70 Machine room
71, 72 Exhaust blower
81, 86 Outer cylinder tube
82, 87 Heat generating body
83, 88 Heater
84 Quartz glass
86a Insulating material
83a, 88a Electric wire
89 Evaporation Tube
A Isolator device
B Leg part
C Chamber
C1 Glass window
C1a to C1d Opening for work
D Device chamber
E Pass box main-body
E1, 3 to 5, 7, 8 Wall part
E2 Carrying-in door
D6 Carrying-out port
G Vial

## Claims

1. A pass box used when an article is to be carried into a work room in which a sterile environment is maintained, having
a pass box main-body having a carrying-in zone into which said article is carried from an outside environment, a decontamination zone in which an outer surface of the carried-in article is decontaminated by a decontamination agent, an aeration zone in which said decontamination agent remaining on said outer surface of said article after a decontamination is removed, and a carrying-out zone from which said article after the aeration is carried out into said work room and a carrying device which carries said article inside said pass body main-body, whereby
said carrying-in zone includes an airtightly sealable carrying-in port through which said article is carried in from said outside environment,
said decontamination zone includes a decontamination-agent applying device which applies a decontamination agent mist to said outer surface of said article having been carried from said carrying-in zone by said carrying device,
said aeration zone includes a clean-air applying device which applies a clean air to a surface of said article having been carried by said carrying device from said decontamination zone,
said carrying-out zone includes a carrying-out port through which said article is carried out by said carrying device to an inside of said work room,
said decontamination-agent applying device includes a compressed-gas generating means which generates a compressed gas, a decontamination-agent supplying means which supplies said decontamination agent, a mixed gas-liquid regulator which mixes said compressed gas and said decontamination agent and regulates a mixed gas-liquid, and a heater which heats said mixed gas-liquid and obtains said decontamination agent mist, and
an amount of said compressed gas supplied from said compressed-gas generating means, an amount of said decontamination agent supplied from said decontamination-agent supply means, and a heating temperature of said heater are controlled, and a temperature of said decontamination agent mist applied to said outer surface of said article is controlled.

2. The pass box for decontamination according to claim 1, wherein
said carrying device operates in a first carrying process, a second carrying process, and a third carrying process,
in said first carrying process, a part of said article which has been carried into said carrying-in zone and has not been decontaminated is supported and is carried from said carrying-in zone to said decontamination zone, and a most part other than the part supported in said decontamination zone is decontaminated,
in said second carrying process, in a state where a part of the part decontaminated in said first carrying process is switched to be supported and is carried from said decontamination zone to said aeration zone, and a part other than the part decontaminated in said first carrying process is decontaminated in said decontamination zone and also a most part other than the part supported in said aeration zone is aerated, and
in said third carrying process, in a state in which a part of the part aerated in said second carrying process is switched to be supported and is carried from said aeration zone to said carrying-out zone, and a part other than the part aerated in said second carrying process is aerated.

3. The pass box for decontamination according to claim 2, wherein
said carrying device is constituted by three units of carrying devices, that is, a first carrying device, a second carrying device, and a third carrying device,
said first carrying device operates in said first carrying process,
said second carrying device operates in said second carrying process, and
said third carrying device operates in said third carrying process.

4. The pass box for decontamination described in claim 2, wherein
said carrying device is constituted by two units of carrying devices, that is, a first carrying device and a second carrying device,
said first carrying device operates in said first carrying process and said third carrying process, and
said second carrying device operates in said second carrying process.

5. The pass box for decontamination according to any one of claims 2 to 4, wherein
said carrying device operates continuously or semi-continuously, and
a total of each operation time of said first carrying process, said second carrying process, and said third carrying process is within 10 minutes.

6. The pass box for decontamination according to any one of claims 1 to 5, wherein
a temperature of said decontamination agent mist is controlled within a range from 30 to 60°C.

7. The pass box for decontamination according to any one of claims 1 to 6, further comprising,
a first exhaust device which exhausts an air in said carrying-in zone and said decontamination zone and a second exhaust device which exhausts an air in said aeration zone and said carrying-out zone, wherein
an exhaust amount of said first exhaust device is set larger than an exhaust amount of said second exhaust device, wherein the air in said carrying-in zone and said decontamination zone does not mix in said aeration zone and said carrying-out zone, and
an air pressure in said pass box main-body is set to a more negative pressure than an air pressure inside said work room, wherein the air in said pass box main-body does not mix inside said work room.

8. The pass box for decontamination according to any one of claims 1 to 7, wherein
said heater includes an introduction port which introduces said mixed gas-liquid, a cylindrical outer cylinder tube, a heat generating body incorporated inside said outer cylinder tube, in parallel to a longitudinal direction of said outer cylinder tube, and an ejection port through which said decontamination agent mist is ejected, and
said mixed gas-liquid is heated while passing between said outer cylinder tube and said heat generating body, the whole or a part of said mixed gas-liquid becomes a decontamination agent gas, and said decontamination agent gas is made into a mist by said compressed gas whose amount is controlled and changed to said decontamination agent mist whose temperature is controlled.

9. The pass box for decontamination according to any one of claims 1 to 7, wherein
said heater includes an introduction port which introduces said mixed gas-liquid, a cylindrical outer cylinder tube, a heat generating body incorporated inside said outer cylinder tube, in parallel to a longitudinal direction of said outer cylinder tube, and an ejection port through which said decontamination agent mist is ejected,
said heat generating body includes a rod-shaped heater disposed in a longitudinal direction thereof and an evaporation tube wound in a spiral state in the longitudinal direction along an outer periphery of said heater, and
said mixed gas-liquid is heated while passing through an inside of said the evaporation tube, the whole or a part of said mixed gas-liquid becomes a decontamination agent gas, and said decontamination agent gas is made into a mist by said compressed gas whose amount is controlled and changed to said decontamination agent mist whose temperature is controlled.

10. The pass box for decontamination described in claim 8 or 9, wherein
said heater includes a mixed gas regulator which mixes said decontamination agent gas and said compressed gas between said heat generating body and said ejection port, and
an amount of said compressed gas introduced into said mixed gas regulator is controlled, wherein said decontamination agent gas is made into a mist by said compressed gas, and is changed to said decontamination agent mist whose temperature is controlled.
